# EUROPÄISCHE PATENTSCHRIFT

(11) **EP 0 949 258 B1**
(45) Veröffentlichungstag und Bekanntmachung des Hinweises auf die Patenterteilung: **31.10.2001**
(21) Anmeldenummer: 99104155.9
(22) Anmeldetag: 02.03.1999
(51) Int. Cl.: C07D 339/04, A61K 31/385

(54) **Alpha-Liponsäure in kristalliner Form**
Alpha-lipoic acid in crystalline form
Acide alpha-lipoique en forme crystalline

(30) Priorität: 11.03.1998 DE 19810336
(43) Veröffentlichungstag der Anmeldung: 13.10.1999
(73) Patentinhaber: ASTA Medica Aktiengesellschaft, 01277 Dresden (DE)
(72) Erfinder: Beisswenger, Thomas, Dr., 01445 Radebeul (DE); Oestreich, Eberhard, 01069 Dresden (DE); Landgraf, Karl-Friedrich, Dr., 01217 Dresden (DE); Laban, Gunter, Dr., 01465 Langebrück (DE); Rischer, Matthias, Dr., 60388 Frankfurt (DE)
(74) Vertreter: Wibbelmann, Jobst, Dr., Dipl.-Chem.

(56) Entgegenhaltungen:
- EP-A- 0 427 247
- EP-A- 0 572 922
- EP-A- 0 593 896
- EP-A- 0 702 953
- ELIEL: "Stereochemistry of Organic Compounds", 1994, WILEY, NEW YORK

## Beschreibung

Alpha-Liponsäure wird in pharmazeutischen Formulierungen sowohl in Infusionslösungen als auch in festen galenischen Formulierungen zur oralen Anwendung verwendet. Hierfür wird synthetisch produzierte, razemische DL-alpha-Liponsäure, die auch als RS-Thioctsäure bezeichnet wird, eingesetzt.

Ein Enantiomer der alpha-Liponsäure, die R-Thioctsäure, kommt als Naturstoff in praktisch allen tierischen und pflanzlichen Zellen vor. Als Coenzym bei der oxidativen Decarboxylierung von alpha-Ketosäuren (z.B. Brenztraubensäure) ist R-Thioctsäure von essentieller Bedeutung. Thioctsäure ist pharmakologisch wirksam und weist antiphlogistische und antinociceptive (analgetische) sowie zytoprotektive Eigenschaften auf. Eine wichtige medizinische Indikation ist die Behandlung der diabetischen Polyneuropathie. Weiterhin findet Thioctsäure Einsatz in der Kosmetik sowie zur Ernährungssupplementierung z. B. aufgrund ihrer antioxidativen Wirkung. Besonders vorteilhaft erscheint dabei die Anwendung der R-Thioctsäure, da diese naturidentisch vorliegt (siehe auch EP 0 572 922 A1), und nur in der natürlichen Form als Cofaktor in den Pyruvat-Dehydrogenase-Komplex eingebaut wird (Oehring et al., Biol. Chem. Hoppe-Seyler 373, 333-335, 1992). Nach neueren Ergebnissen (Baur et al., Klin. Wochenschr. 1991, 69(15), 722-4) kann Thioctsäure möglicherweise Bedeutung bei der Bekämpfung der durch HIV-1-und HTLV IIIB-Viren bedingten Krankheit erlangen.

Bei den reinen optischen Isomeren der Thioctsäure (R- und S-Form, d.h. R-Thioctsäure und S-Thioctsäure) ist im Gegensatz zum Razemat das R-Enantiomer vorwiegend antiphlogistisch und das S-Enantiomer vorwiegend antinociceptiv wirksam (siehe auch EP 0 427 247 A2). Zur Erzielung einer selektiven Wirkung ist die Herstellung und Anwendung der reinen Enantiomere daher von großer Wichtigkeit.

Zur gezielten Herstellung der reinen Enantiomere, der R- oder S-Thioctsäure, sind eine Reihe von Verfahren bekannt, die in der Regel eine enantioselektive Synthesestufe zur Erzeugung einer geeigneten chiralen Vorstufe oder Zwischenstufe beinhalten. Alle bisher bekannten Verfahren erfordern einen hohen synthetischen Aufwand, bzw. Anstrengungen zur Abreicherung des unerwünschten Enantiomers, und haben bisher keinen Einsatz in technischem Maßstab ermöglicht.

Der Schmelzbereich der reinen Enantiomere der Thioctsäure (47 bis 49°C) liegt gegenüber der razemischen Verbindung (58-61°C) niedriger. Bei der Herstellung fester galenischer Formulierungen, die in der Regel unter Pressung oder Kompaktierung verlaufen, ist die Anwendung von Druck auf das Material unerläßlich, so daß einerseits eine Erwärmung und andererseits ein Schmelzen der Thioctsäure stattfindet. Konzentrierte Lösungen von Thioctsäure oder ihre Schmelzen polymerisieren sofort und lassen sich durch Abkühlen nicht mehr in eine kristalline Form überführen.

Dieser Effekt ist bei reinen Enantiomeren der Thioctsäure aufgrund des niedrigeren Schmelzpunktes stark ausgeprägt. Für den an sich wünschenswerten therapeutischen Einsatz der reinen Enantiomere wurde die Verwendung von basischen Salzen vorgeschlagen (siehe auch EP 702 953 A2).

"Aus EP 0 593 896 A1 ist eine kristalline RS-Thioctsäure und enantiomerenreine kristalline R- und S-Thioctsäure bekannt, die durch das Intensitätsverhältnis der Reflexionslinien bei 2θ= 18° und 2θ= 22° und den Extinktionskoeffizienten einer Thioctsäurelösung charakterisiert ist und die durch eine Umkristallisation in einem Lösungsmittel bzw.
Lösungsmittelgemisch mit der Dielektrizitätskonstante epsilon zwischen 2,5 und 5,5 erhalten werden kann."

Aufgabe der vorliegenden Erfindung ist es, Thioctsäure herzustellen, die das erwünschte Enantiomer angereichert enthält, und dabei andererseits eine Modifikation oder Form aufzufinden, die sich aufgrund ihrer physikalischen Eigenschaften bei ihrer Verarbeitung möglichst weitgehend wie die razemische Thioctsäure verhält.

Liegt bei der Herstellung der Thioctsäure durch ein geeignetes synthetisches Herstellungsverfahren bereits ein Enantiomer angereichert, aber noch nicht vollständig vor, so erhält man überraschenderweise bei der Kristallisation aus geeigneten Lösungsmitteln Thioctsäure, die das vorherrschende Enantiomer angereichert enthält, sich aber nicht wie die entsprechenden Feststoffmischungen des kristallinen Razemats mit der reinen kristallinen R- oder S-Thioctsäure verhalten. Die gebildete, neuartige Modifikation zeigt ein Röntgenpulverdiffraktogramm, das denen des Razemats, der reinen Enantiomere bzw. Mischungen derselben nicht entspricht

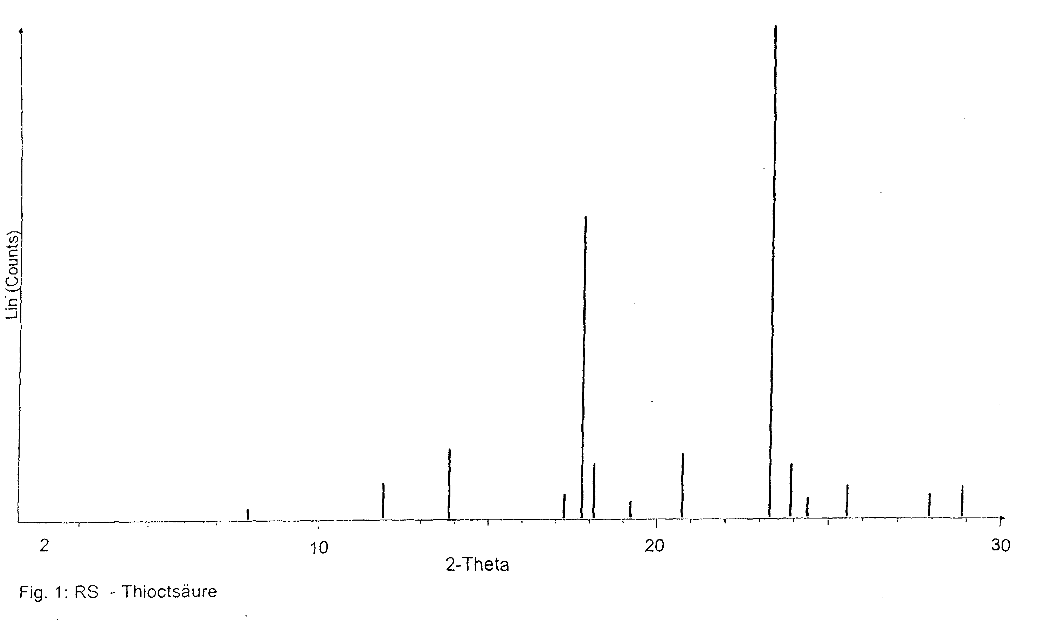

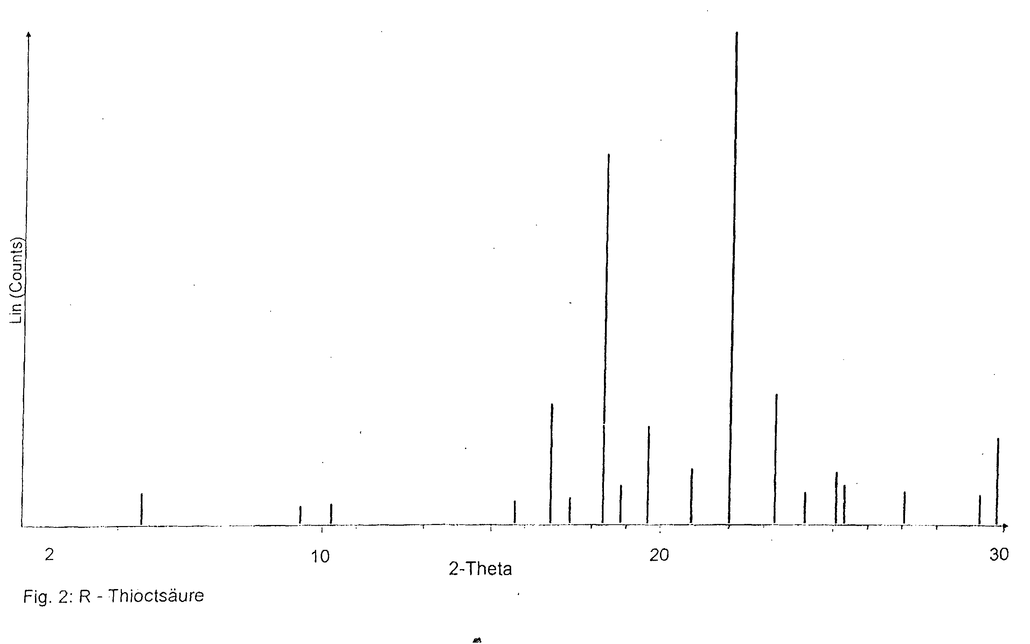

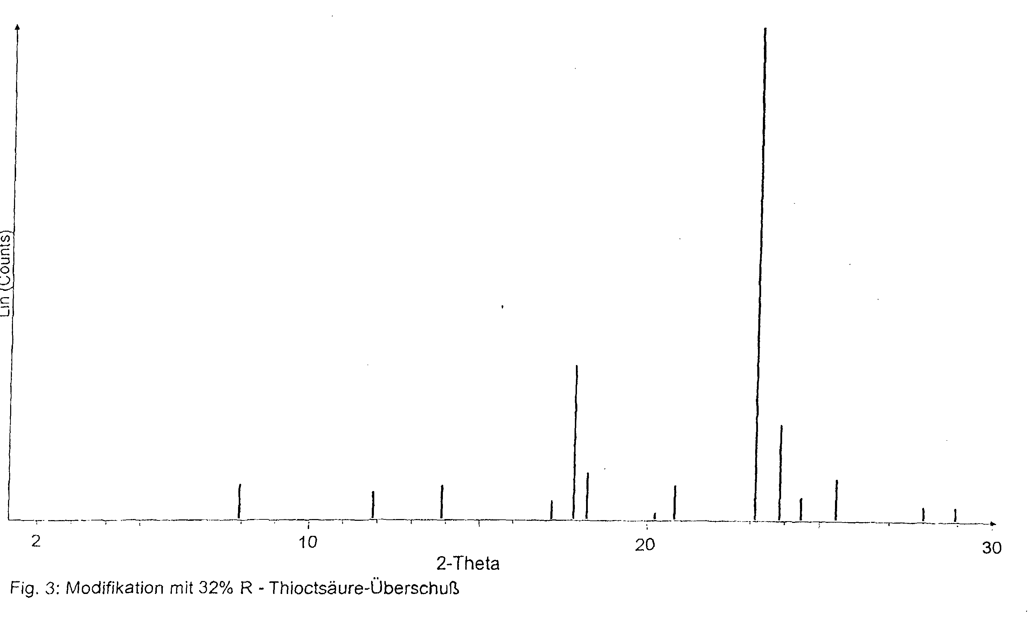

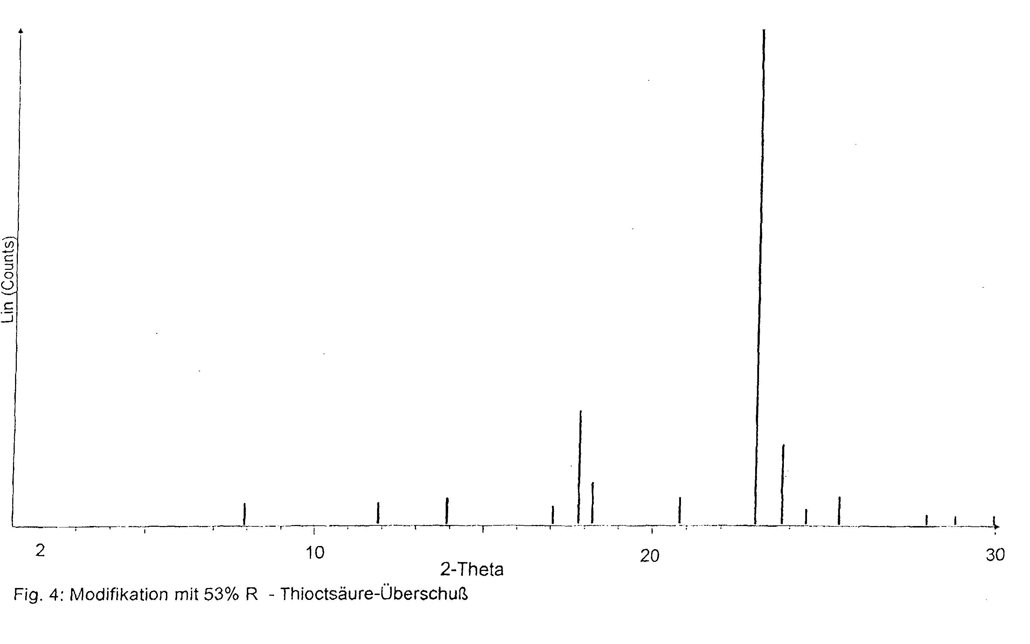

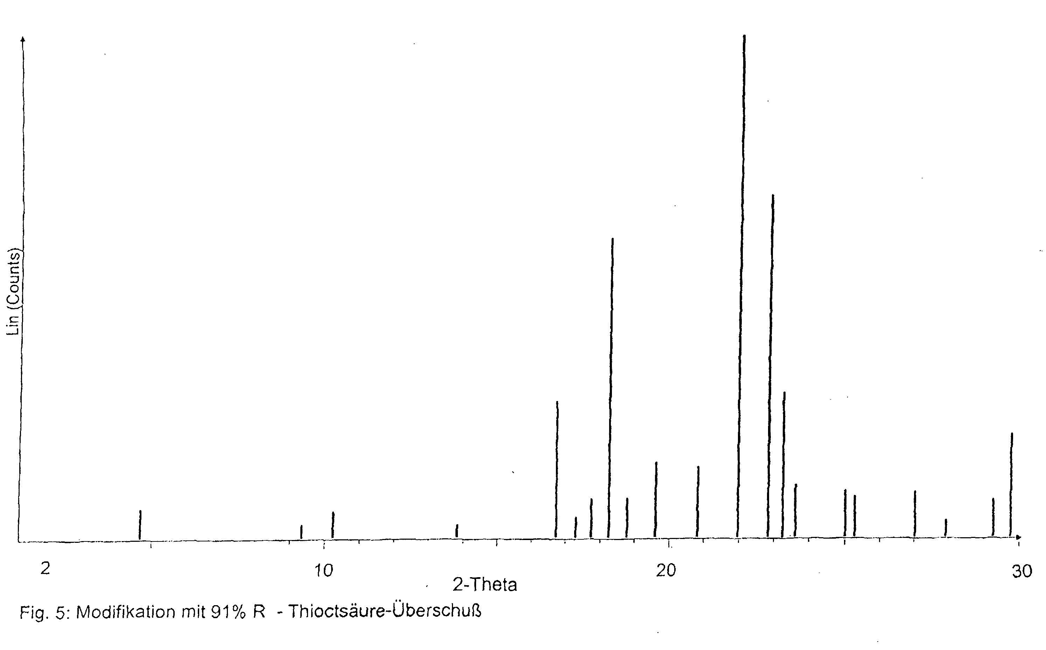

Die vorliegende Erfindung bezieht sich auf Thioctsäure mit überwiegendem Anteil eines Enantiomeren, vorzugsweise mit einem Enantiomerverhältnis von 60:40 bis 97:3, die in einer neuartigen Modifikation vorliegt. In den Figuren 1 und 2 sind die typischen, literaturbekannten Röntgendiffraktogrammaufnahmen der razemischen RS-Thioctsäure sowie der reinen R-Thioctsäure dargestellt. Weiterhin zeigen die Figuren 3 bis 5 Röntgenpulverdiffraktogramme, die von kristallisierter Thioctsäure stammen, die aus Lösungen von an reinen Enantiomeren angereicherter Thioctsäure hergestellt werden. In Figur 3 wird eine Thioctsäure mit einem Anteil R-Enantiomer von 66 % und 34 % S-Enantiomer, in Figur 4 mit einem Anteil R-Enantiomer von 76 % und 24 % S-Enantiomer und in Figur 5 eine Thioctsäure mit einem Anteil R-Enantiomer von 95 % mit 5 % S-Enantiomer abgebildet. Die Röntgendiffraktogramme wurden in Reflexionsstellung aufgenommen.
Überraschenderwiese zeigt die erfindungsgemäße Thioctsäure einen Schmelzbereich von 48 bis 59°C, der von dem erwarteten eutektischen Schmelzbereich von 44 bis 48 °C abweicht. Weiterhin ermöglicht sie eine bevorzugte galenische Verarbeitung und weist gegenüber dem reinen Enantiomer eine bessere Temperaturstabilität auf.

Die Kristallisation der Thioctsäure kann in einem geeigneten organischen Lösungsmittel vorgenommen werden. Beispiele für organische Lösungsmittel, die auch Wasser enthalten können, sind u.a. aliphatische Kohlenwasserstoffe mit einer Kohlenstoffkettenlänge zwischen 3 und 10 Kohlenstoffatomen, aromatische Kohlenwasserstoffe, die flüssig sind, Ester aus aliphatischen oder cycloaliphatischen Carbonsäuren mit 2 bis 6 Kohlenstoffatomen und aliphatischen oder cycloaliphatischen Alkoholen mit 1 bis 6 Kohlenstoffatomen, aliphatischen oder cycloaliphatischen Alkoholen mit 1 bis 6 Kohlenstoffatomen, Ether und Glycolether oder homogene Gemische der genannten Lösungsmittel. Besonders bevorzugte Lösemittel sind Essigsäureethylester, Hexan, Cyclohexan, Pentan, Heptan, Diisopropylether, Toluol, Ethanol und deren homogene Gemische.

Die Reinheit und Zusammensetzung der erhaltenen Thioctsäuren wurde mittels Analyse auf einer chiralen HPLC-Säule ermittelt. Die Bestimmung der Schmelzbereiche erfolgte mittels Differential Scanning Calorimetry (DSC) mit einer Aufheizrate von 2°K/min.
Die vorliegende Erfindung ermöglicht es, die Enantiomere der Thioctsäure in angereicherter Form, die auf einfache und wirtschaftliche Weise aus Lösungen derselben kristallin und rein erhalten werden können, für verschiedene Anwendungen zugänglich zu machen.

Die Erfindung wird durch nachfolgende Beispiele näher erläutert.

### Beispiel 1

In einem Gemisch aus 960 ml Cyclohexan und 240 ml Essigsäureethylester wurden bei 40° C 41,2 g racemische Thioctsäure aufgelöst und anschließend 12,0 g (100 mmol) S-(-)-α-Methylbenzylamin langsam zugetropft.
Dann wurde auf 25° C abgekühlt, der Niederschlag abgesaugt und mit Cyclohexan-Essigsäureethylester-Gemisch nachgewaschen. Zum Filtrat wurden 660 ml Wasser zugegeben und bei Raumtemperatur mit ca. 10 %iger Salzsäure ein pH-Wert von 1 - 1,5 eingestellt. Die Phasen wurden getrennt und die Wasserphase noch einmal mit 60 ml Cyclohexan-Essigsäureethylester-Gemisch extrahiert.
Die vereinigten organischen Phasen wurden unter Vakuum auf ca. 1/5 des ursprünglichen Volumens eindestilliert.
Der erhaltene Destillationsrückstand wurde auf -5° bis -10° C abgekühlt und zur Kristallisation nachgerührt.
Der Niederschlag wurde abfiltriert, gewaschen und getrocknet.
Es wurden 20,4 g Thioctsäure in der neuen Modifikation als Erstkristallisat erhalten. Der Gehalt an R-(+)-Thioctsäure betrug 69,0 %.

### Beispiel 2

Eine Lösung, die in einem Gemisch aus 225 ml Cyclohexan und 25 ml Essigsäureethylester 20,0 g R-(+)-Thioctsäure und 5,0 g S-(-)-Thioctsäure enthielt, wurde von 35 bis 40 °C auf -5 bis -10 °C abgekühlt, filtriert und getrocknet. Es wurden 17,3 g Thioctsäure in der neuen Modifikation als Erstkristallisat erhalten. Der Gehalt an R-(+)-Thioctsäure betrug 75,6 % mit einem Schmelzbereich von 49 bis 54 °C.

### Beispiel 3

Eine Lösung, die in einem Gemisch aus 225 ml Cyclohexan und 25 ml Essigsäureethylester 11,7 R-(+)-Thioctsäure und 5,0 g S-(-)-Thioctsäure enthielt, wurde von 35 bis 40 °C auf -5 bis -10 °C abgekühlt, filtriert und getrocknet. Es wurden 12,0 g Thioctsäure in der neuen Modifikation als Erstkristallisat erhalten. Der Gehalt an R-(+)-Thioctsäure betrug 65,8 % mit einem Schmelzbereich von 54 bis 58 °C.

### Beispiel 4

Eine Lösung, die in einem Gemisch aus 225 ml Cyclohexan und 25 ml Essigsäureethylester 95,0 g R-(+)-Thioctsäure und 5,0 g S-(-)-Thioctsäure enthielt, wurde von 35 bis 40 °C auf -5 bis -10 °C abgekühlt, filtriert und getrocknet Es wurden 87,1 g Thioctsäure in der neuen Modifikation als Erstkristallisat erhalten. Der Gehalt an R-(+)-Thioctsäure betrug 93,5 % mit einem Schmelzbereich von 45 bis 47 °C.

### Beispiel 5

Eine Lösung, die in 80 ml Diisopropylether 4,0 g R-(+)-Thioctsäure und 16,0 g S-(-)-Thioctsäure enthielt, wurde von 35 bis 40 °C auf -5 bis -10 °C abgekühlt, filtriert und getrocknet. Es wurden 14,5 g Thioctsäure in der neuen Modifikation als Erstkristallisat erhalten. Der Gehalt an S-(-)-Thioctsäure betrug 75,8 % mit einem Schmelzbereich von 50 bis 56 °C.

### Beispiel 6

Eine Lösung, die in 80 ml Diisopropylether 16,6 g R-(+)-Thioctsäure und 3,4 g S-(-)-Thioctsäure enthielt, wurde von 35 bis 40 °C auf -5 bis -10 °C abgekühlt, filtriert und getrocknet. Es wurden 13,5 g Thioctsäure in der neuen Modifikation als Erstkristallisat erhalten. Der Gehalt an R-(+)-Thioctsäure betrug 78,8 % mit einem Schmelzbereich von 48 bis 54 °C.

### Beispiel 7

Eine Lösung, die in einem Gemisch aus 200 ml n-Hexan und 57 ml Essigsäureethylester 17,5 g R-(+)-Thioctsäure und 2,5 g S-(-)-Thioctsäure enthielt, wurde von 35 bis 40 °C auf -5 bis -10 °C abgekühlt, filtriert und getrocknet. Es wurden 13,5 g Thioctsäure in der neuen Modifikation als Erstkristallisat erhalten. Der Gehalt an R-(+)-Thioctsäure betrug 82,6 % mit einem Schmelzbereich von 47 bis 52 °C.

### Beispiel 8

Eine Lösung, die in einem Gemisch aus 24 ml Toluol und 6 ml n-Heptan 19,5 g R-(+)-Thioctsäure und 0,5 g S-(-)-Thioctsäure enthielt, wurde von 35 bis 40 °C auf -5 bis -10 °C abgekühlt, filtriert und getrocknet. Es wurden 13,0 g Thioctsäure in der neuen Modifikation als Erstkristallisat erhalten. Der Gehalt an R-(+)-Thioctsäure betrug 94,5 % mit einem Schmelzbereich von 45 bis 48 °C.

### Beispiel 9

Eine Lösung, die in einem Gemisch aus 135 ml Cyclohexan und 15 ml Essigsäureethylester 3,0 g R-(+)-Thioctsäure und 7,0 g S-(-)-Thioctsäure enthielt, wurde von 35 bis 40 °C auf -5 bis -10 °C abgekühlt, filtriert und getrocknet. Es wurden 8,5 g Thioctsäure in der neuen Modifikation als Erstkristallisat erhalten. Der Gehalt an S-(-)-Thioctsäure betrug 67,8 % mit einem Schmelzbereich von 53 bis 58 °C.

## Patentansprüche

1. Thioctsäure mit überwiegendem Anteil eines Enantiomeren, **dadurch gekennzeichnet, daß** die Röntgenpulverdiffraktogramme einen charakteristischen Reflex im Bereich 23,4 bis 22,7° 2theta(Cu) aufweisen, der sich mit zunehmendem Enantiomergehalt in Richtung der kleineren Winkelwerte verschiebt.

2. Thioctsäure gemäß Anspruch 1, **dadurch gekennzeichnet, daß** ein Enantiomergehalt an R- oder S-Thioctsäure von 60 bis 97 % vorliegt.

3. Thioctsäure gemäß Ansprüchen 1 und 2 **dadurch gekennzeichnet, daß** ein Schmelzbereich von 48 bis 59 °C vorliegt.

4. Verfahren zur Herstellung kristalliner Thioctsäure gemäß einem der Ansprüche 1 bis 3, **dadurch gekennzeichnet, daß** Thioctsäuren, die ein Enantiomer in angereicherter Form enthalten, durch Kristallisation aus geeigneten Lösungsmitteln erhalten werden.

5. Verfahren gemäß Anspruch 4, **dadurch gekennzeichnet, daß** organische Lösungsmittel verwendet werden.

6. Verwendung von Thioctsäure nach einem der vorstehenden Ansprüche 1 bis 3 zur Herstellung von Arzneimittelformulierungen oder von Ergänzungsmitteln in Kosmetik oder Ernährung.

## Claims

1. Thioctic acid having a predominant content of one enantiomer, **characterized in that** the X-ray powder diffractograms have a characteristic reflection in the range of 23.4 to 22.7° 2theta(Cu), which shifts in the direction of the smaller angular values with increasing enantiomer content.

2. Thioctic acid according to Claim 1, **characterized in that** an enantiomer content of R- or S-thioctic acid of 60 to 97% is present.

3. Thioctic acid according to Claims 1 and 2, **characterized in that** the melting range is from 48 to 59°C.

4. Process for the preparation of crystalline thioctic acid of Claims 1 to 3, **characterized in that** thioctic acids which contain one enantiomer in enriched form are obtained by crystallization from suitable solvents.

5. Process according to Claim 4, **characterized in that** organic solvents are used.

6. Use of thioctic acid according to one of the preceding Claims 1 to 3 for the manufacture of pharmaceutical formulations or of supplements in cosmetics or nutrition.

## Revendications

1. Acide thiooctique avec une proportion prédominante d'un énantiomère,
**caractérisé en ce que**
les diagrammes de diffraction en poudre aux rayons X possèdent une réflexion caractéristique dans la zone de 23,4 à 22,7° 2theta(Cu) qui se déplace avec une teneur croissante en énantiomère en direction des valeurs d'angle plus petites.

2. Acide thiooctique conformément à la revendication 1,
**caractérisé en ce qu'**
il a une teneur en énantiomère en acide (R) ou (S) thiooctique allant de 60 à 97 %.

3. Acide thiooctique selon l'une quelconque des revendications 1 et 2,
**caractérisé en ce qu'**
il présente une zone de fusion allant de 48 à 59°C.

4. Procédé de préparation d'acide thiooctique cristallin selon l'une quelconque des revendications 1 à 3,
**caractérisé en ce que**
les acides thiooctiques qui renferment un énantiomère sous forme enrichie, sont obtenus par cristallisation à partir de solvants appropriés.

5. Procédé de préparation selon la revendication 4,
**caractérisé en ce qu'**
on utilise des solvants organiques.

6. Utilisation de l'acide thiooctique selon l'une quelconque des revendications 1 à 3, en vue de la préparation de formulations de médicament ou d'agents de complément en cosmétique ou dans l'alimentation.
